# EUROPEAN PATENT APPLICATION

(11) **EP 4 173 576 A1**
(43) Date of publication of application: **03.05.2023**
(21) Application number: 21832207.1
(22) Date of filing: 16.03.2021
(51) Int. Cl.: A61B 17/00, A61B 34/30, B25J 18/06

(54) **CONTINUOUS BODY INSTRUMENT AND SURGICAL ROBOT**

(30) Priority: 30.06.2020 CN 202010617404; 30.06.2020 CN 202010618751; 30.06.2020 CN 202010623370; 30.06.2020 CN 202010618747
(71) Applicant: Beijing Surgerii Technology Co., Ltd., Beijing 100192 (CN)
(72) Inventor: XU, Kai, Beijing 100192 (CN); LIU, Xu, Beijing 100192 (CN)
(74) Representative: karo IP
(86) International application number: PCT/CN2021/080946
(87) International publication number: WO 2022/001186

(57) **Abstract**

A continuum instrument (10, 20, 30, 40), relating to the field of medical instruments, and comprising at least one proximal continuum (111, 111') and at least one distal continuum (112, 112'). The proximal continuum (111, 111') comprises a proximal base disk (1111, 1111'), a first proximal stop disk (1112, 1112'), a second proximal stop disk (1114, 1114'), a plurality of proximal structural backbones (1116, 1116') and a plurality of proximal drive backbones (1113), with proximal ends of the plurality of proximal drive backbones (1113) being fixedly connected to the second proximal stop disk (1114, 1114'), and the plurality of proximal drive backbones (1113) passing through the first proximal stop disk (1112, 1112') and having distal ends fixedly connected to the proximal base disk (1111, 1111'). The distal continuum (112, 112') comprises a distal stop disk (1122) and a plurality of distal structural backbones (1123), the plurality of distal structural backbones (1123) being connected to or integrally formed with the plurality of proximal structural backbones (1116, 1116'), and distal ends of the plurality of distal structural backbones (1123) being fixedly connected to the distal stop disk (1122). By means of driving the second proximal stop disk (1114, 1114') to move, the proximal structural backbones (1116, 1116') and the distal structural backbones (1123) are driven to bend so as to drive the distal continuum (112, 112') to bend. Thus, the structural backbones can be prevented from being directly pushed and pulled.

## Description

### Cross Reference to Related Applications

The present application claims the right of priority of the Chinese patent application No. 2020106174040, filed on June 30, 2020, and entitled "Integrally Drivable Flexible Continuum Structure and Flexible Robot Arm", the Chinese patent application No. 2020106187515, filed on June 30, 2020, and entitled "Surgical Tool Drive Transmission System Based on Planar Motion Mechanism, and Surgical Robot", the Chinese patent application No. 2020106233706, filed on June 30, 2020, and entitled "Surgical Tool Driving System, and Surgical Robot", and the Chinese patent application No. 2020106187479, filed on June 30, 2020, and entitled "Surgical Tool Drive Transmission System, and Surgical Robot Including Same", which are incorporated herein by reference in their entirety.

### Technical Field

The present disclosure relates to the field of medical instruments, and in particular to a continuum instrument and a surgical robot.

### Background Art

Minimally invasive procedures cause less injury to patients and faster postoperative recovery, and have been of great significance in surgery. In a minimally invasive procedure, surgical instruments, including surgical tools and visual lighting modules, all enter the human body through an incision or a natural orifice and then reach a surgical site to perform a surgical operation. In an existing surgical instrument, a distal structure is mainly composed of multiple rods hinged in series, and is driven by a pulling force from a steel wire rope so that the surgical instrument can bend at a hinged joint. Since the steel wire rope must be maintained in a continuous tension state by means of a pulley, this driving method can hardly achieve further miniaturization of the surgical instrument and further improvement of kinematic performance of the instrument.

In contrast to a traditional rigid kinematic chain which achieves a bending motion by means of mutual rotation at joints, a flexible continuum structure can achieve continuous bending deformation, and thus the flexible continuum structure is widely used in the research and development of medical instruments such as flexible manipulators, endoscopes and controllable catheters, and new-type special equipment such as industrial deep-cavity detection endoscopes and flexible mechanical arms.

Generally, in an existing continuum structure, a drive wire in the continuum structure is directly pushed and pulled by means of a drive mechanism so that the continuum structure can bend in any direction. However, with the stricter requirements for a continuum structure, such as high precision, fast response, high flexibility of bending, and good stability, the existing drive structures gradually no longer satisfy the above requirements. In addition, in the existing driving method, the motion is performed by means of directly pushing and pulling a drive wire, and thus when there is a large number of drive wires, the number of drive mechanisms will also increase accordingly, making the structure complex.

### Summary of the Invention

In some embodiments, the present disclosure provides a continuum instrument, comprising: at least one proximal continuum comprising a proximal base disk, a first proximal stop disk, a second proximal stop disk, a plurality of proximal structural backbones and a plurality of proximal drive backbones, with proximal ends of the plurality of proximal drive backbones being fixedly connected to the second proximal stop disk, the plurality of proximal drive backbones passing through the first proximal stop disk, and distal ends of the plurality of proximal drive backbones being fixedly connected to the proximal base disk; at least one distal continuum comprising a distal stop disk and a plurality of distal structural backbones, the plurality of distal structural backbones being connected to or integrally formed with the plurality of proximal structural backbones, with distal ends of the plurality of distal structural backbones being fixedly connected to the distal stop disk.

In some embodiments, the present disclosure provides a surgical robot, comprising at least one surgical trolley, at least one positioning arm, and at least one surgical instrument, wherein the at least one surgical instrument comprises at least one continuum instrument as described above and an end device disposed at a distal end of the continuum instrument; and the at least one positioning arm is movably disposed on the at least one surgical trolley, and at least one surgical instrument is disposed at a distal end of the at least one positioning arm.

### Brief Description of the Drawings

In order to explain the technical solutions in the embodiments of the present disclosure more clearly, the accompanying drawings used in the description of the embodiments will be briefly introduced below. Obviously, the accompanying drawings in the following description only show some of the embodiments of the present disclosure, and for those of ordinary skill in the art, other embodiments would also have been obtained from the contents of the embodiments of the present disclosure and these accompanying drawings without involving any inventive effort.
Fig. 1 shows a schematic structural diagram of a continuum instrument according to some embodiments of the present disclosure;
Fig. 2 shows a partial schematic structural diagram of another continuum instrument according to some embodiments of the present disclosure;
Fig. 3 shows a schematic structural diagram of a drive transmission mechanism according to some embodiments of the present disclosure;
Fig. 4 shows a top view of the drive transmission mechanism shown in Fig. 3 according to some embodiments of the present disclosure;
Fig. 5 shows a schematic structural diagram of a third connecting rod of a drive transmission mechanism shown in Fig. 3 according to some embodiments of the present disclosure;
Fig. 6 shows a schematic structural diagram of a fourth connecting rod of the drive transmission mechanism shown in Fig. 3 according to some embodiments of the present disclosure;
Fig. 7 shows a partial schematic structural diagram of another continuum instrument according to some embodiments of the present disclosure;
Fig. 8 shows a partial schematic structural diagram of the continuum instrument shown in Fig. 7 according to some embodiments of the present disclosure;
Fig. 9 shows a schematic structural diagram of the drive transmission mechanism shown in Fig. 7 according to some embodiments of the present disclosure;
Fig. 10 shows a partial schematic structural diagram of the drive transmission mechanism shown in Fig. 9 according to some embodiments of the present disclosure;
Fig. 11 shows a partial schematic structural diagram of the drive transmission mechanism shown in Fig. 9 according to some embodiments of the present disclosure;
Fig. 12 shows a partial schematic structural diagram of another continuum instrument according to some embodiments of the present disclosure;
Fig. 13 shows a schematic structural diagram of a drive transmission mechanism shown in Fig. 11 according to some embodiments of the present disclosure;
Fig. 14 shows a partial schematic structural diagram of the drive transmission mechanism shown in Fig. 12 according to some embodiments of the present disclosure;
Fig. 15 shows a partial schematic structural diagram of the drive transmission mechanism shown in Fig. 12 according to some embodiments of the present disclosure;
Fig. 16 shows a partial schematic structural diagram of the drive transmission mechanism shown in Fig. 12 according to some embodiments of the present disclosure;
Fig. 17 shows a partial schematic structural diagram of another continuum instrument according to some embodiments of the present disclosure;
Fig. 18 shows a partial schematic structural diagram of another continuum instrument according to some embodiments of the present disclosure;
Fig. 19 shows a partial schematic structural diagram of another continuum instrument according to some embodiments of the present disclosure; and
Fig. 20 shows a partial schematic structural diagram of a surgical robot according to some embodiments of the present disclosure.

### Detailed Description of the Embodiments

In order to clarify the technical problem to be solved, the technical solutions used and the technical effects achieved in the present disclosure in a better way, the technical solutions of embodiments of the present disclosure will be described in further detail below in conjunction with the accompanying drawings. Obviously, the embodiments described are merely exemplary embodiments, rather than all the embodiments of the present disclosure.

In the description of the present disclosure, it should be noted that the orientation or position relationships indicated by the terms "center", "upper", "lower", "left", "right", "vertical", "horizontal", "inner", "outer", etc. are based on the orientation or position relationships shown in the accompanying drawings and are merely for ease of description of the present disclosure and simplification of the description, rather than indicating or implying that the devices or elements referred to must have a specific orientation or be constructed and operated in a specific orientation, and thus cannot be construed as limiting the present disclosure. Moreover, the terms "first" and "second" are merely used for the illustrative purpose, and should not be construed as indicating or implying the relative importance. In the description of the present disclosure, it should be noted that the terms "mounting", "connecting", "connection" and "coupling" should be appreciated in a generalized sense, unless otherwise explicitly specified and defined, and for example, may be a fixed connection or a detachable connection, may be a mechanical connection or an electrical connection, may be a direct connection or an indirect connection via an intermediate medium, and may be communication between the interiors of two components. For those of ordinary skill in the art, the specific meanings of the terms mentioned above in the present disclosure should be construed according to specific circumstances. The present disclosure defines the end close to an operator (e.g., a surgeon) as a proximal end or portion or a rear end or portion, and the end close to a patient undergoing surgery as a distal end or portion or a front end or portion. Those skilled in the art will appreciate that the embodiments of the present disclosure can be used in medical instruments or surgical robots, and can also be used in other non-medical devices.

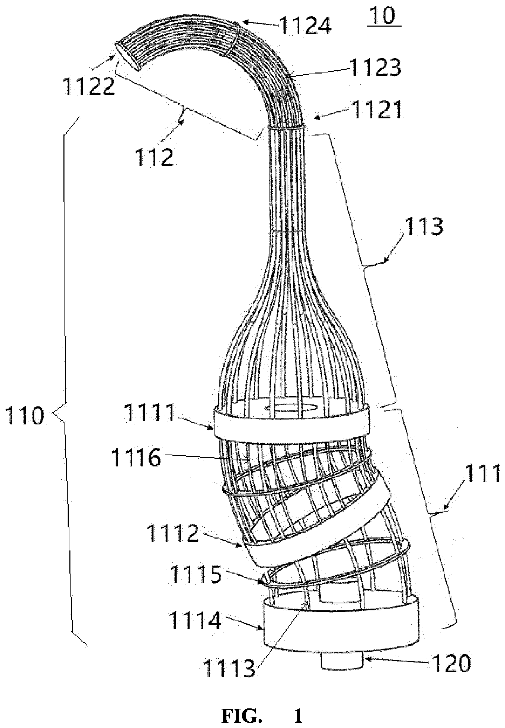

Fig. 1 shows a continuum instrument 10 according to some embodiments of the present disclosure. As shown in Fig. 1, the continuum instrument 10 may include a flexible continuum structure 110. The flexible continuum structure 110 may include at least one proximal continuum 111 located at a proximal end, and at least one distal continuum 112 located at a distal end. The proximal continuum 111 may include a proximal base disk 1111, a first proximal stop disk 1112, proximal drive backbones 1113, a second proximal stop disk 1114, and proximal structural backbones 1116. The proximal base disk 1111, the first proximal stop disk 1112, and the second proximal stop disk 1114 are arranged at intervals. Proximal ends of the plurality of proximal drive backbones 1113 are fixedly connected to the second proximal stop disk 1114. The plurality of proximal drive backbones 1113 pass through the first proximal stop disk 1112, and have distal ends fixedly connected to the proximal base disk 1111. Proximal ends of the plurality of proximal structural backbones 1116 are fixedly connected to the first proximal stop disk 1112.

The distal continuum 112 may include a distal base disk 1121, a distal stop disk 1122, and distal structural backbones 1123. The distal base disk 1121 and the distal stop disk 1122 are arranged at an interval, and the distal base disk 1121 is adjacent to the proximal base disk 1111. The plurality of distal structural backbones 1123 are connected to or integrally formed with the plurality of proximal structural backbones 1116, and pass through the proximal base disk 1111 and the distal base disk 1121.

As shown in Fig. 1, in some embodiments, the flexible continuum structure 110 may further include a structural backbone guide tube bundle 113. A proximal end of the structural backbone guide tube bundle 113 is fixedly connected to the proximal base disk 1111, and a distal end of the structural backbone guide tube bundle 113 is fixedly connected to the distal base disk 1121. The plurality of distal structural backbones 1123 or the plurality of proximal structural backbones 1116 sequentially pass through the proximal base disk 1111, the structural backbone guide tube bundle 113 and the distal base disk 1121. The structural backbone guide tube bundle 113 may guide and constrain the plurality of distal structural backbones 1123 or the plurality of proximal structural backbones 1116 located between the proximal base disk 1111 and the distal base disk 1121.

In some embodiments, the continuum instrument 10 may further include a drive connection part 120. A distal end of the drive connection part 120 is connected to the second proximal stop disk 1114, and a proximal end of the drive connection part 120 includes an input end. The input end is used for being driven by the drive transmission mechanism such that the second proximal stop disk 1114 moves to drive the first proximal stop disk 1112 to turn to allow the proximal structural backbones 1116 and the distal structural backbones 1123 to be pushed and pulled so as to achieve the bending of the distal continuum 112 in a space in different directions. In some embodiments, the drive connection part 120 may include at least one movable joint (not shown). The at least one movable joint may include at least one of a cylindrical pair, a moving pair, and a rotating pair. A distal end of the at least one movable joint is connected to the second proximal stop disk 1114 by means of at least one of a cylindrical pair, a moving pair, a rotating pair, and fixed connection. A proximal end of the at least one movable joint forms an input end, and the input end and the output end of the drive transmission mechanism may be connected by means of at least one of a cylindrical pair, a moving pair, a rotating pair, and fixed connection. It should be appreciated that in the present disclosure, the cylindrical pair may rotate or move, the moving pair can move only, and the rotating pair can rotate only. The second proximal stop disk 1114 can slide up and down or rotate relative to the drive connection part 120 or the drive connection part 120 can slide up and down or rotate relative to the output end of the drive transmission mechanism, so as to allow the proximal continuum 111 to generate a parasitic motion sliding in the axial direction (slipping up and down) and a bending motion in any direction (rotation) during bending. The parasitic motion may prevent the distal continuum 112 from generating an axial telescoping motion during bending that may cause wrinkling or excessive stretching of an envelope that covers the outer periphery of the distal continuum 112 to affect the service life of the cover.

In some embodiments, the continuum instrument 10 may include a drive transmission mechanism. An output end of the drive transmission mechanism is connected to the second proximal stop disk 1114 and used for driving the second proximal stop disk 1114 to move, such that the first proximal stop disk 1112 is driven to turn, so as to drive the distal continuum 112 to bend by means of the proximal drive backbones 1113, the proximal structural backbones 1116 and the distal structural backbones 1123. In some embodiments, the output end of the drive transmission mechanism may include a sliding pin, and the second proximal stop disk 1114 may include a guide hole. A sliding pin is disposed in the guide hole and is connected to the second proximal stop disk 1114 by means of at least one of a cylindrical pair, a moving pair, and a rotating pair. In some embodiments, the output end of the drive transmission mechanism may include an annular shaft sleeved on the outer periphery of the second proximal stop disk 1114, and is connected to the second proximal stop disk 1114 by means of at least one of a cylindrical pair, a moving pair, and a rotating pair.

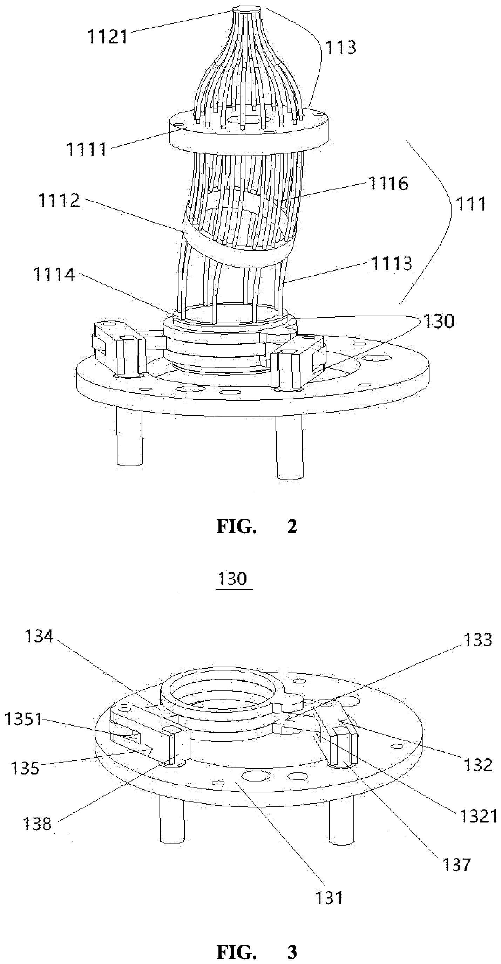

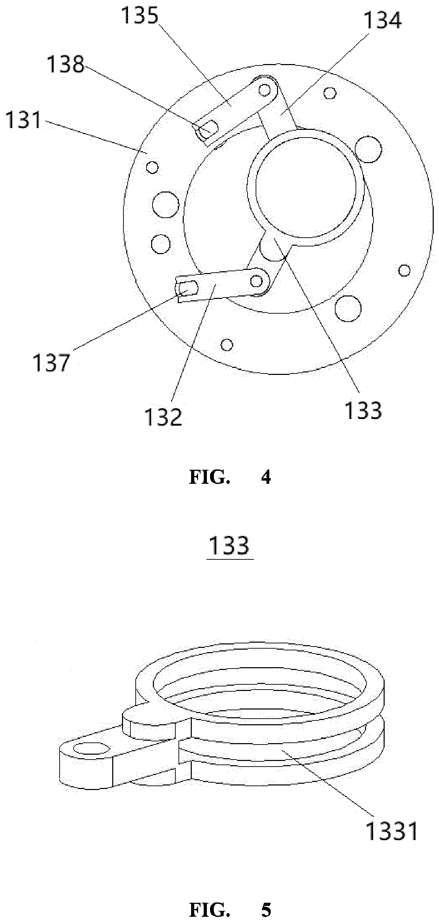

In some embodiments, the continuum instrument 10 may include a drive transmission mechanism. An output end of the drive transmission mechanism may perform a planar motion. Figs. 2 shows a partial schematic structural diagram of the continuum instrument 10 according to some embodiments of the present disclosure including a drive transmission mechanism 130. Fig. 3 and Fig. 4 respectively show a schematic structural diagram and a top view of the drive transmission mechanism 130 according to some embodiments of the present disclosure. In some embodiments, as shown in Figs. 2-4, the drive transmission mechanism 130 may include a planar link mechanism. The planar link mechanism may include a first connecting rod 131, a second connecting rod 132, a third connecting rod 133, a fourth connecting rod 134, a fifth connecting rod 135, a first input shaft 137, and a second input shaft 138. The first connecting rod 131 is fixedly disposed, and the first input shaft 137 and the second input shaft 138 are rotatably disposed on the first connecting rod 131. One end of the second connecting rod 132 is fixedly connected to the first input shaft 137, and the other end of the second connecting rod 132 is hinged to one end of the third connecting rod 133. One end of the fifth connecting rod 135 is fixedly connected to the second input shaft 138, and the other end of the fifth connecting rod 135 is hinged to one end of the fourth connecting rod 134. The other end of the fourth connecting rod 134 is movably connected to the other end of the third connecting rod 133. The other end of the third connecting rod 133 or the fourth connecting rod 134 forms the output end of the drive transmission mechanism, and the output end is connected to the second proximal stop disk 1114, for example, by means of a cylindrical pair, such that the output end of the drive transmission mechanism 130 and the second proximal stop disk 1114 can slide and rotate in an axial direction of the output end. The second proximal stop disk 1114 may be moved in any direction in a horizontal plane by means of a planar link mechanism. In some embodiments, the output end of the drive transmission mechanism 130 may also be connected to the input end of the drive connection part 120.

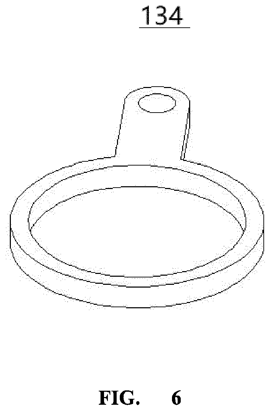

In some embodiments, as shown in Figs. 3 and 4, the first connecting rod 131 may include an arc-shaped link rod formed by a base, and the second connecting rod 132 and the fifth connecting rod 135 may be straight, and one end of the second connecting rod 132 and one end of the fifth connecting rod 135 are respectively fixedly connected to the first input shaft 137 and the second input shaft 138 and thus can rotate along with the rotation of the first input shaft 137 and the second input shaft 138. Figs. 5 and 6 respectively show schematic structural diagrams of the third connecting rod and the fourth connecting rod of the drive transmission mechanism 130 according to some embodiments of the present disclosure. In some embodiments, as shown in Figs. 5 and 6, one end of the third connecting rod 133 may comprise a straight rod, and the other end comprises a circular ring-shaped shaft. The circular ring-shaped shaft is provided with an arc-shaped groove 1331 in a circumferential direction. One end of the fourth connecting rod 134 may comprise a straight rod, and the other end includes a circular ring having a size corresponding to the size of the arc-shaped groove 1331. The circular ring of the fourth connecting rod 134 are embedded into the arc-shaped grooves 1331 so as to be rotatable with each other. The circular ring-shaped shaft of the third connecting rod 133 and the circular ring of the fourth connecting rod 134 cooperate to form the output end (e.g., an annular shaft) of the drive transmission mechanism 130. The circular ring-shaped shaft and the circular ring are sleeved on the outer periphery of the second proximal stop disk 1114 (or the outer periphery of the input end), for example, they are connected by means of a cylindrical pair such that the output end of the drive transmission mechanism 130 and the second proximal stop disk 1114 can slide and rotate in the axial direction of the output end. In some embodiments, the output end of the drive transmission mechanism 130 may also include a sliding pin, and the second proximal stop disk 1114 may include a guide hole. The sliding pin is disposed in the guide hole and is slidable and rotatable with the second proximal stop disk 1114 in the axial direction of the sliding pin.

As shown in Fig. 3, the second connecting rod 132 may include an opening 1321, and the fifth connecting rod 135 may include an opening 1351. One end of the third connecting rod 133 is located in the opening 1321 and is hinged to the other end of the second connecting rod 132. One end of the fourth connecting rod 134 is located in the arc-shaped opening 1351 and is hinged to the other end of the fifth connecting rod 135. The configuration of the openings may facilitate the rotation of the third connecting rod 133 and the fourth connecting rod 134. It should be appreciated that the connecting rods may also be hinged to each other on the surfaces of or outside the connecting rods, which can also achieve the rotation of the connecting rods.

Thus, as shown in Figs. 2-6, when the first input shaft 137 and/or the second input shaft 138 are driven to rotate, the second connecting rod 132 and the fifth connecting rod 135 are driven to rotate, so as to drive the third connecting rod 133 and the fourth connecting rod 134 to rotate and then drive the output end (e.g., the circular ring-shaped shaft of the third connecting rod 133 and the circular ring of the fourth connecting rod 134) of the planar five-connecting-rod mechanism to move in a plane. By means of the output end, the second proximal stop disk 1114 can be driven to move in the plane, such that the proximal base disk 1111 and the second proximal stop disk 1114 are displaced and have the axes thereof no longer coincident. In this way, the plurality of proximal drive backbones 1113 can be driven to bend, and then the proximal continuum 111 is bent to drive the first proximal stop disk 1112 to turn, such that the plurality of proximal structural backbones 1116 which have ends fixed to the first proximal stop disk 1112 are pushed and pulled so as to push and pull the plurality of distal structural backbones 1123 fixed to (e.g., uniformly distributed on) the first proximal stop disk 1112. Since the overall length of the proximal structural backbones 1116 and the distal structural backbones 1123 is substantially unchanged, resulting in a corresponding change in the length of the distal structural backbones 1123 in the distal continuum 112, the distal continuum 112 is thus driven to bend corresponding to (e.g., in the same direction, in an opposite direction, or angled with) the proximal continuum 111. The second proximal stop disk 1114 is driven to move so as to drive the proximal drive backbones 1113 to bend, such that the first proximal stop disk 1112 turns to push and pull the proximal structural backbones 1116 and the distal structural backbones 1123, instead of directly pushing and pulling the proximal structural backbones 1113 and the distal structural backbones 1123. A large number of structural backbones can be driven without being limited by the number of the drive transmission mechanisms, achieving a compact structure and very high reliability and flexibility.

It should be noted that the proportions of bending of the proximal continuum 111 and the distal continuum 112 are respectively inversely proportional to the corresponding distribution radii of the proximal structural backbones 1116 and the distal structural backbones 1123 in the two continua (in this embodiment, the proximal structural backbones 1116 in the proximal continuum 111 and the distal structural backbones 1123 in the distal continuum 112 are respectively distributed in a circumferential direction, may be distributed on a circumference or in a peripheral direction of a rectangular, polygonal, elliptical or other shape, and may be in a uniform or non-uniform distribution). Therefore, when in use, the distribution radii of the proximal structural backbones 1116 and the distal structural backbones 1123 in the proximal continuum 111 and the distal continuum 112 may be respectively adjusted to meet the actual requirements of the proportion of bending.

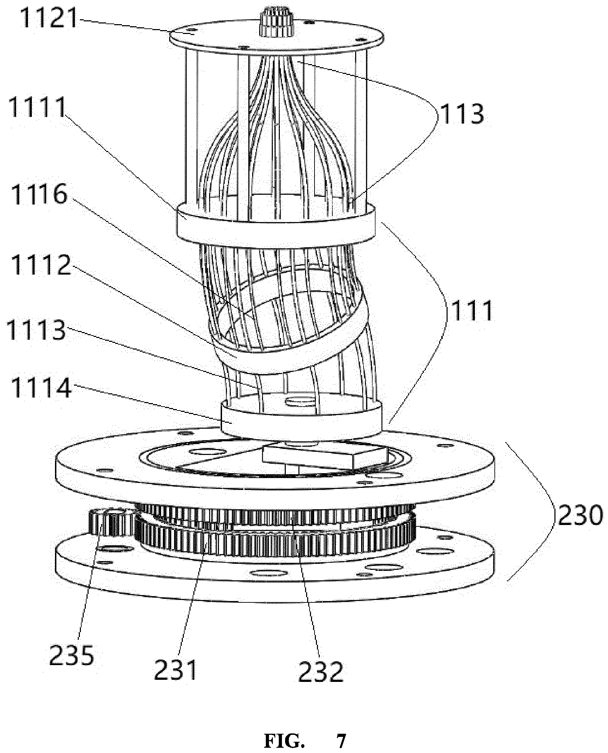

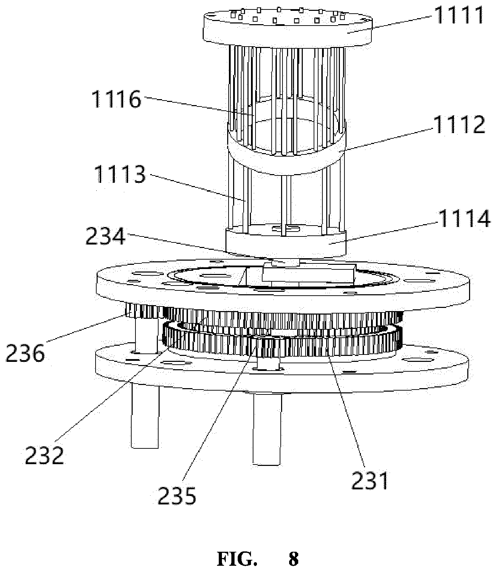

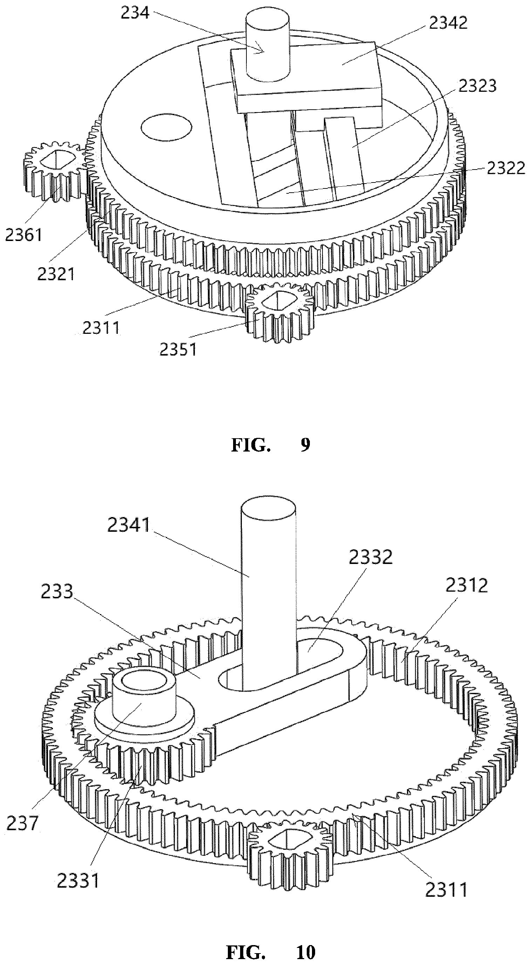

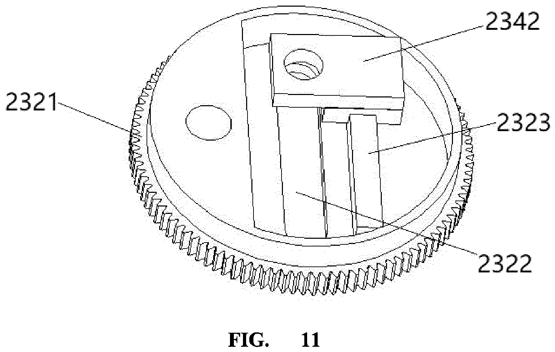

Figs. 7 and 8 respectively show a partial schematic structural diagram of a continuum instrument 10 of the drive transmission mechanism 230 according to some embodiments of the present disclosure, and Fig. 9 shows a schematic structural diagram of the drive transmission mechanism 230 according to some embodiments of the present disclosure. Figs. 10 and 11 show partial schematic structural diagrams of the drive transmission mechanism 230 according to some embodiments of the present disclosure. In some embodiments, as shown in Figs. 7-10, the drive transmission mechanism 230 may include a gear and sliding groove mechanism, and the gear and sliding groove mechanism may include a first rotatable member 231, a moving member 233, a second rotatable member 232, and a sliding assembly 234. The first rotatable member 231 may be used for being driven by a first drive member 235 to rotate around its own center of rotation. The moving member 233 may be used for being driven by the rotation of the first rotatable member 231 to rotate around its own center of rotation, and the center of rotation of the moving member 233 is offset from the center of rotation of the first rotatable member 231. The moving member 233 is provided with a first sliding guide portion 2332, as shown in Fig. 10. The second rotatable member 232 is coaxially disposed with the first rotatable member 231 and may be used for being driven by the second drive member 236 to rotate relative to the first rotatable member 231, and the second rotatable member 232 is provided with a second sliding guide portion 2322, as shown in Fig. 9. As shown in Figs. 9 and 10, the sliding assembly 234 is slidably connected to the first sliding guide portion 2332 and the second sliding guide portion 2322 to slide along the first sliding guide portion 2332 and the second sliding guide portion 2322. The sliding assembly 234 is connected to the second proximal stop disk 1114, for example, by means of a cylindrical pair, such that the sliding assembly 234 of the drive transmission mechanism 230 and the second proximal stop disk 1114 can slide and rotate in the axial direction of the output end. In some embodiments, the sliding assembly 234 may also be connected to the input end of the drive connection part 120.

In some embodiments, as shown in Fig. 9, the second rotatable member 232 may be arranged above the first rotatable member 231 in an overlapping manner, and the two rotatable members are rotatable relative to each other. As shown in Fig. 9, in some embodiments, the first rotatable member 231 may include, for example, a first driven gear 2311. The first drive member 235 may include a first driving gear 2351. The second rotatable member 232 may include, for example, a second driven gear 2321. The second drive member 236 may include a second driving gear 2361. The first driving gear 2351 meshes with the first driven gear 2311, the second driving gear 2361 meshes with the first driven gear 2311, and the second driven gear 2321 is arranged above the first driven gear 2311 in an overlapping manner. The first driving gear 2351 may be driven by a drive electric motor to drive the first driven gear 2311 to rotate. The second driving gear 2361 may be driven by the drive electric motor to drive the second driven gear 2321 to rotate, and the first driven gear 2311 and the second driven gear 2321 are rotatable relative to each other. In some embodiments, the first rotatable member 231 and the second rotatable member 232 may respectively include a first gear and a second gear, the first drive member 235 and the second drive member 236 may include a drive electric motor (or a motor), and the first gear and the second gear can be respectively driven by the drive electric motor to rotate relative to each other. In some embodiments, the transmission mode of the first rotatable member 231 and the second rotatable member 232 may also be other transmission modes, such as belt pulley transmission or sprocket transmission.

In some embodiments, as shown in Fig. 10, the moving member 233 may include a meshing portion 2331. The meshing portion 2331 is used for meshing with the first rotatable member 231. In some embodiments, the moving member 233 may be a link rod having at least one end portion which is circular arc-shaped. The meshing portion 2331 may include teeth disposed on an outer peripheral surface of the circular arc-shaped end portion. An inner peripheral surface of the first rotatable member 231 (e.g., the first driven gear 2311) is provided with inner ring teeth 2312, and the teeth of the outer peripheral surface of the circular arc-shaped end portion mesh with the inner ring teeth 2312 of the first rotatable member 231 so that, when the first rotatable member 231 rotates, the moving member 233 is driven to rotate with the first rotatable member 231. It should be noted that in some embodiments, a further gear (not shown) may be provided in the first rotatable member 231, and the further gear coaxially and synchronously rotates with the first rotatable member 231, and the teeth on the outer peripheral surface of the moving member 233 mesh with the further gear, so that the moving member 233 can be driven by the first rotatable member 231 to rotate.

In some embodiments, as shown in Figs. 9-11, the moving member 233 is provided with a first sliding guide portion 2332. The first sliding guide portion 2332 may include a first sliding groove disposed in the lengthwise direction of the moving member 233. The second rotatable member 232 (e.g., the second driven gear 2321) is provided with a second sliding guide portion 2322, and the second sliding guide portion 2322 may include a second sliding groove formed along the diameter of the second rotatable member 232. The sliding assembly 234 may include a sliding pin 2341. The sliding pin 2341 is slidably disposed in the first sliding groove and the second sliding groove, so that the sliding pin 2341 can move along the first sliding groove and/or the second sliding groove. The distal end of the sliding pin 2341 forms the output end of the drive transmission mechanism 230 and is connected to the second proximal stop disk 1114, for example, by means of a cylindrical pair, such that the sliding pin 2341 and the second proximal stop disk 1114 can slide and rotate in the axial direction of the sliding pin 2341. In some embodiments, as shown in Fig. 9, the sliding assembly 234 may further include a sliding block 2342 fixedly connected to or integrally formed with the sliding pin 2341. The second rotatable member 232 may be provided with a sliding rail 2323 parallel to the second sliding groove, and the sliding block 2342 is slidably disposed on the sliding rail 2323. For example, the sliding block 2342 and the sliding rail 2323 may be in a groove-type cooperation. A proximal end of the sliding pin 2341 is slidably disposed in the first sliding groove, and a distal end thereof passes through the first sliding groove and the second sliding groove and is then fixedly connected to the sliding block 2342. The distal end of the sliding pin 2341 may pass through the sliding block 2342 and is then connected to the second proximal stop disk 1114 or is connected to the second proximal stop disk 1114 by means of the sliding block 2342. The cooperation of the sliding block 2342 and the sliding rail 2323 can guide the motion of the sliding pin 2341, so that the sliding pin 2341 can move more smoothly.

In some embodiments, the first sliding guide portion 2332 may include a first sliding rail (not shown) disposed in the lengthwise direction of the moving member 233, and the second sliding guide portion 2322 includes a second sliding rail disposed along the diameter of the second rotatable member 232. The sliding assembly 234 may include a first sliding block, a second sliding block, and a sliding pin. The first sliding block is slidably disposed on the first sliding rail, the second sliding block is slidably disposed on the second sliding rail, one of the first sliding block and the second sliding block is disposed to be movably connected to the sliding pin, and the other of the first sliding block and the second sliding block is disposed to be fixedly connected to or integrally formed with the sliding pin. The sliding pin can also slide along the first sliding rail and/or the second sliding rail. It should also be appreciated that one of the first sliding guide portion 2332 and the second sliding guide portion 2322 may include a sliding rail, the other of the first sliding guide portion 2332 and the second sliding guide portion 2322 may include a sliding groove, and the sliding assembly 234 may include a sliding block and a sliding pin fixedly connected to or integrally formed with the sliding block, the sliding pin being disposed in the sliding groove, and the sliding block being slidably disposed on the sliding rail.

In some embodiments, as shown in Fig. 10, the drive transmission mechanism 230 may further include a rotating shaft 237. A proximal end of the rotating shaft 237 may be fixedly connected to or integrally formed with the moving member 233, the rotating shaft 237 may be located at the end close to the circular arc-shaped end portion, and a distal end of the rotating shaft 237 may be rotatably disposed on the second rotatable member 232, so that the rotating shaft 237 may rotate relative to the second rotatable member 232; and the axis of rotation of the rotating shaft 237 is offset from the center of rotation of the second rotatable member 232, so that when the first driven gear 2311 rotates at any angle, the second sliding groove can always intersect the first sliding groove, with the sliding pin 2341 being located at the point of intersection of the two sliding grooves.

Thus, as shown in Figs. 7-11, when the first driving gear 2351 drives the first driven gear 2311 located at the lower layer to rotate while the second driving gear 2361 located at the upper layer remains stationary, the moving member 233 meshing with the first driven gear 2311 is driven to rotate around the rotating shaft 237, so as to drive the sliding pin 2341 located in the first sliding groove and the second sliding groove to move. By means of the limiting cooperation between the second sliding groove and the first sliding groove, the sliding pin 2341 can move linearly along the second sliding groove, so as to drive the second proximal stop disk 1114 to move by means of the sliding pin 2341, such that the proximal base disk 1111 and the second proximal stop disk 1114 are displaced and have the axes thereof no longer coincident. The plurality of proximal drive backbones 1113 are driven to bend, and then the proximal continuum 111 is bent to drive the first proximal stop disk 1112 to turn so as to push and pull the plurality of proximal structural backbones 1116 which have ends fixed to the first proximal stop disk 1112, so that the distal structural backbones 1123 drive the distal continuum 112 to bend corresponding to (e.g., in the same direction, in an opposite direction, or angled with) the proximal continuum 111, which can achieve the bending of the distal continuum 112 in the space along a specific bending plane. The distance that the sliding pin 2341 or the sliding block 2342 moves along the second sliding groove can be adjusted so as to adjust the degree of bending of the proximal continuum 111 to adjust the degree of bending of the distal continuum 112. In the case where the second driving gear 2361 drives the second driven gear 2321 to rotate so that the first driving gear 2351 drives the first driven gear 2311 to rotate, and the second driven gear 2321 and the first driven gear 2311 synchronously rotate in the same direction (e.g., at a same speed), the position of the sliding pin 2341 in the first sliding groove and the second sliding groove does not change, but the azimuth angle of the sliding pin 2341 in the plane of rotation of the driven gears changes (e.g., the sliding pin 2341 moves in a circular motion) so as to change the orientation of the bending plane of the sliding pin 2341. After the proximal continuum 111 is bent, the push and pull action generated on the proximal structural backbones 1116 by the first proximal stop disk 1112 is transferred to the distal end of the distal continuum 112 by means of the distal structural backbones 1123, so as to achieve the bending of the distal continuum 112 in a space along different bending planes. The second driven gear 2321 and the first driven gear 2311 are driven cooperatively to adjust the degree of bending of the proximal continuum 111 along the specific bending plane and the bending thereof in different bending planes, so as to achieve the bending of the distal continuum 112 in the space in any direction.

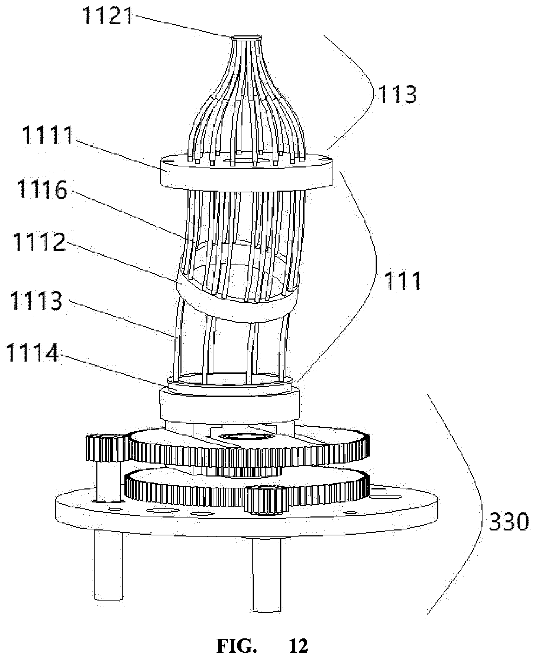

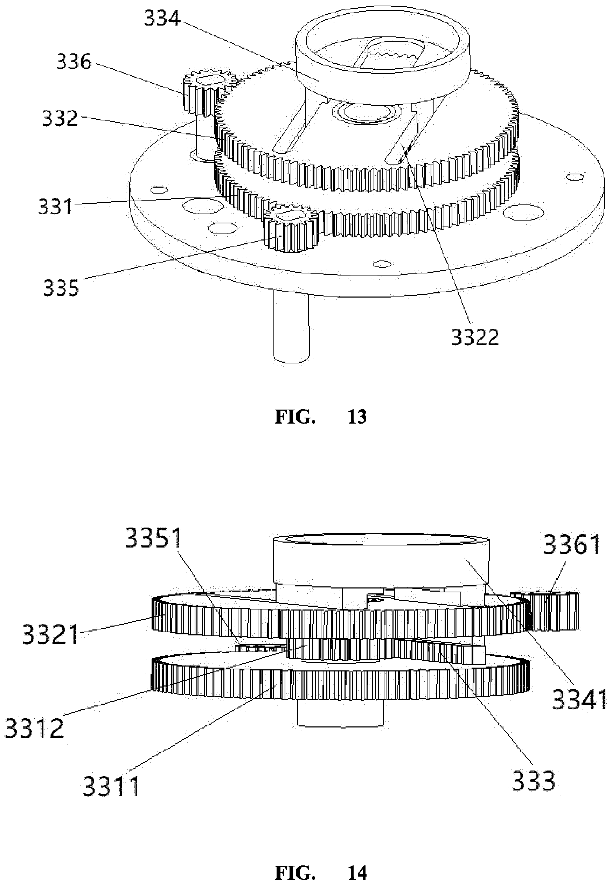

Figs. 12 shows a partial schematic structural diagram of the continuum instrument 10 according to some embodiments of the present disclosure including a drive transmission mechanism 330. Fig. 13 shows a schematic structural diagram of the drive transmission mechanism 330 according to some embodiments of the present disclosure. Figs. 14-16 show partial schematic structural diagrams of the drive transmission mechanism 330 according to some embodiments of the present disclosure. In some embodiments, as shown in Figs. 12 and 13, the drive transmission mechanism 330 may include a gear and rack mechanism, which may include a first rotatable member 331, a second rotatable member 332, and a moving assembly. The first rotatable member 331 may be used for being driven by a first drive member 335 to rotate, and the second rotatable member 332 is coaxially disposed with the first rotatable member 331 and may be used for being driven by a second drive member 336 to rotate relative to the first rotatable member 331. The second rotatable member 332 is provided with a sliding guide portion 3322, as shown in Fig. 13. At least a portion of the moving assembly is slidably disposed on the sliding guide portion 3322, at least another portion of the moving assembly is disposed to be able to move linearly along with the rotation of the first rotatable member 331, a distal end of the moving assembly forms the output end of the drive transmission mechanism 330, the output end is connected to the second proximal stop disk 1114, for example, by means of a cylindrical pair, such that the output end of the drive transmission mechanism 330 and the second proximal stop disk 1114 can slide and rotate in the axial direction of the output end. In some embodiments, the distal end of the moving assembly may also be connected to the drive connection part 120, and drives the second proximal stop disk 1114 to move by means of the drive connection part 120.

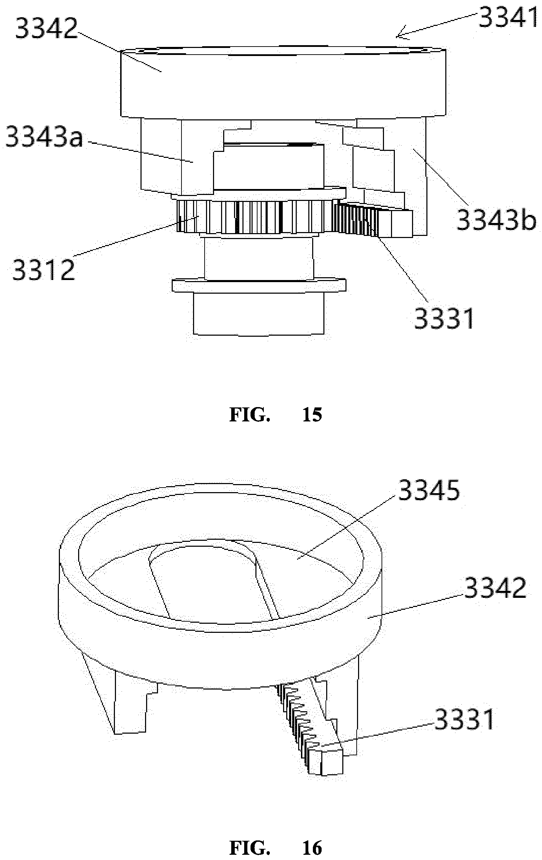

In some embodiments, as shown in Fig. 13, the second rotatable member 332 may be arranged above the first rotatable member 331 in an overlapping manner, and the two rotatable members are rotatable relative to each other. As shown in Figs. 14 and 15, in some embodiments, the first rotatable member 331 may include a meshing gear 3312 and a first driven gear 3311 coaxially and fixedly connected to each other, and the meshing gear 3312 is located at a distal side of the first driven gear 3311. The first drive member 335 may include a first driving gear 3351. The second rotatable member 332 may include, for example, a second driven gear 3321, and the second drive member 336 may include a second driving gear 3361. The first driving gear 3351 meshes with the first driven gear 3311, the second driving gear 3361 meshes with the second driven gear 3321, and the second driven gear 3321 is arranged above the meshing gear 3312 in an overlapping manner. The first driving gear 3351 may be driven by a drive electric motor to drive the first driven gear 3311 to rotate, the second driving gear 3361 may be driven by the drive electric motor to drive the second driven gear 3321 to rotate, and the first driven gear 3311 and the second driven gear 3321 are rotatable relative to each other. In some embodiments, the first rotatable member 331 may include the meshing gear 3312 and a first gear coaxially and fixedly connected to each other, the second rotatable member 332 may include a second gear, the first drive member 335 and the second drive member 336 may include a drive electric motor (or a motor), and the first gear and the second gear can be respectively driven by the drive electric motor to rotate relative to each other. In some embodiments, the transmission mode of the first rotatable member 331 and the second rotatable member 332 may also be other transmission modes, such as belt pulley transmission or sprocket transmission.

As shown in Figs. 13 and 14, in some embodiments, the moving assembly may include a sliding portion 334 and a meshing portion 333 which are fixedly connected to each other or integrally formed. The sliding portion 334 is slidably disposed on the sliding guide portion 3322 and is then guided by the sliding guide portion 3322 to slide linearly relative to the second rotatable member 332 (e.g., the second driven gear 3321), and the meshing portion 333 is disposed to mesh with the first rotatable member 331 (e.g., the first driven gear 3311) and then move linearly along with the rotation of the first rotatable member 331. As shown in Figs. 15 and 16, in some embodiments, the meshing portion 333 may include a rack 3331. The rack 3331 may mesh with the meshing gear 3312. It should be appreciated that the rack 3331 may be a spur rack, and the meshing gear 3312 may be a spur gear. In addition, the rack 3331 may also be a helical rack, and the meshing gear 3312 may also be a helical gear. In some embodiments, the sliding portion 334 may include a sliding block 3341, and the sliding guide portion 3322 may include at least one sliding groove provided in a direction parallel to the diameter of the second rotatable member 332, the sliding block 3341 is slidably disposed in the sliding groove, and one side of the sliding block 3341 passes through the sliding groove and is fixedly connected to the rack 3331. The rack 3331 may be driven by the meshing gear 3312 to move linearly so as to drive the sliding block 3341 to move along the sliding groove. In some embodiments, as shown in Fig. 13, the sliding guide portion 3322 may include a pair of sliding grooves symmetrically disposed along the diameter of the second rotatable member 332. As shown in Figs. 15, the sliding block 3341 may include a sliding block body 3342 and sliding block side wings 3343a and 3343b proximally extending from two ends of the sliding block body 3342. Inner sides of the sliding block side wings 3343a and 3343b are provided with snap-fit steps for cooperation with the sliding grooves. The sliding block side wing 3343b passes through the sliding groove and is then fixedly connected to the rack 3331. As shown in Fig. 16, the sliding block body 3342 is provided with a connecting groove 3345, and the connecting groove 3345 is sleeved on the outer periphery (or an outer proximal periphery) of the second proximal stop disk 1114, for example, by means of a cylindrical pair, such that the sliding block body 3342 and the second proximal stop disk 1114 can slide and rotate in the axial direction of the connecting groove 3345. In some embodiments, the connecting groove 3345 may be circular, corresponding to the shape of the second proximal stop disk 1114. In some embodiments, the connecting groove 3345 may also be in another shape, such as rectangle and polygon. The shape of the outer periphery of the second proximal stop disk 1114 matches the shape of the inner periphery of the connecting groove 3345, and the second proximal stop disk and the connecting groove can be connected by means of the moving pair. It should be appreciated that the sliding guide portion 3322 may also include at least one sliding rail (or sliding rod) disposed in a direction parallel to the diameter of the second rotatable member 332, and the sliding block 3341 is slidably disposed on the sliding rail. For example, the sliding block 3341 and the sliding rail may be in a groove-type cooperation.

Thus, as shown in Figs. 12-16, when the first driving gear 3351 drives the first driven gear 3311 located at the lower layer to rotate while the second driving gear 3361 located at the upper layer remains stationary, the meshing gear 3312 fixedly connected to the first driven gear 3311 is driven to rotate so as to drive the rack 3331 meshing with the meshing gear 3312 to move linearly. The sliding block 3341 fixedly connected to the rack 3331 is driven by the rack 3331 to move in the sliding groove of the second driven gear 3321, so as to drive the second proximal stop disk 1114 to move in a horizontal plane, such that the proximal base disk 1111 and the second proximal stop disk 1114 are displaced and have the axes thereof no longer coincident. The plurality of proximal drive backbones 1113 are driven to bend, and then the proximal continuum 111 is bent to drive the first proximal stop disk 1112 to turn so as to push and pull the plurality of proximal structural backbones 1116 which have ends fixed to the first proximal stop disk 1112, so that the distal structural backbones 1123 drive the distal continuum 112 to bend corresponding to (e.g., in the same direction, in an opposite direction, or angled with) the proximal continuum 111, which can achieve the bending of the distal continuum 112 in the space along a specific bending plane. In the case where the second driving gear 3361 drives the second driven gear 3321 to rotate so that the first driving gear 3351 drives the first driven gear 3311 to rotate, and the second driven gear 3321 and the first driven gear 3311 synchronously rotate in the same direction (e.g., at a same speed), the position of the sliding block 3341 on the second rotatable member 332 does not change, but the azimuth angle of the sliding block 3341 in a plane of rotation of the driven gear changes (e.g., the sliding block moves in a circular motion in a plane), so as to change the orientation of the bending plane of the sliding block 3341. After the proximal continuum 111 is bent, the push and pull action generated on the proximal structural backbones 1116 by the first proximal stop disk 1112 is transferred to the distal continuum 112 by means of the distal structural backbones 1123, so as to achieve the bending of the distal continuum 112 in a space along different bending planes. The second driven gear 3321 and the first driven gear 3311 are driven cooperatively to adjust the degree of bending of the proximal continuum 111 along the specific bending plane and the bending thereof in different bending planes, so as to achieve the bending of the distal continuum 112 in the space in any direction.

As shown in Fig. 1, in some embodiments, the proximal continuum 111 may further include at least one proximal retaining disk 1115 disposed between the proximal base disk 1111 and the second proximal stop disk 1114, and the plurality of proximal drive backbones 1113 or proximal structural backbones 1116 sequentially pass through the at least one proximal retaining disk 1115. As shown in Fig. 1, in some embodiments, the distal continuum 112 may further include at least one distal retaining disk 1124 disposed between the distal base disk 1121 and the distal stop disk 1122, and the plurality of distal structural backbones 1123 pass through the at least one distal retaining disk 1124. The proximal retaining disk 1115 and the distal retaining disk 1124 are used for respectively supporting the structural backbones in the radial directions of the proximal drive backbones 1113, the proximal structural backbones 1116 and the distal structural backbones 1123, such that the proximal drive backbones 1113, the proximal structural backbones 1116 and the distal structural backbones 1123 remain in a parallel state during the bending transformation, which can prevent the proximal drive backbones 1113, the proximal structural backbones 1116 and the distal structural backbones 1123 from destabilizing during the bending motion. In some embodiments, at least one tube bundle retaining disk (not shown) is disposed on the structural backbone guide tube bundle 113, a proximal end of the structural backbone guide tube bundle 113 is fixedly connected to the proximal base disk 1111, and a distal end of the structural backbone guide tube bundle 113 passes through the at least one tube bundle retaining disk and is then fixedly connected to the distal base disk 1121.

In some embodiments, the proximal drive backbone 1113, the proximal structural backbone 1116 and the proximal structural backbone 1116 may include elastic wires or tubes made of a hyperelastic material, for example, may be made of an elastic metallic material having high strength and high toughness, such as a nickel-titanium alloy. The structural backbone guide tube bundle 113 may include a plurality of thin tubes made of a steel material to form a steel tube bundle.

In some embodiments, the continuum instrument 20 may include at least two continuum instruments 10 in the embodiments described above. In some embodiments, the continuum instrument 20 includes at least two continuum instruments 10 connected in series or in parallel.

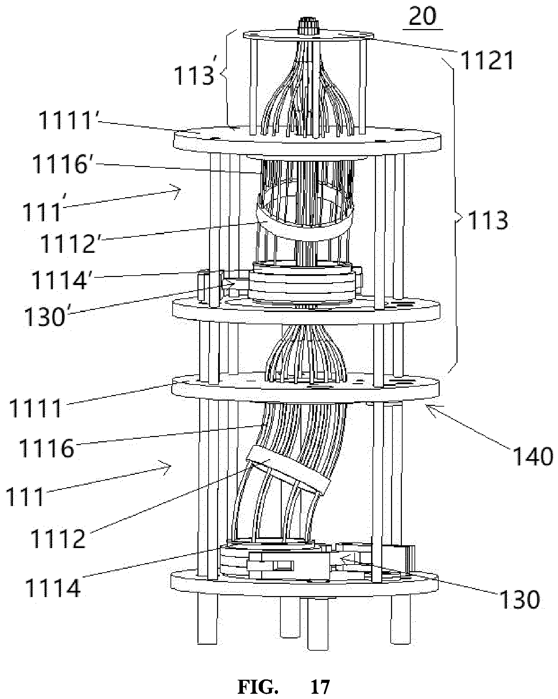

Fig. 17 shows a partial schematic structural diagram of the continuum instrument 20 according to some embodiments of the present disclosure. As shown in Fig. 17, in some embodiments, the continuum instrument 20 further includes a support 140. At least two proximal continua 111, 111' are respectively disposed at the proximal end and the distal end of the support 140, and the proximal base disks 1111, 1111' of the at least two proximal continua 111, 111' are respectively fixedly connected to or integrally formed with the support 140. The proximal ends of at least two structural backbone guide tube bundles 113, 113' are fixedly connected to the proximal base disks 1111, 1111' of the respective proximal continua 111, 111'. The distal end of the structural backbone guide tube bundle 113 located at the proximal end passes through the support 140 and the second proximal stop disk 1114', the first proximal stop disk 1112' and the proximal base disk 1111' of the proximal continuum 111' located at the distal end, and converges into one bundle at the same distal base disk 1121 together with the proximal continuum 111' located at the distal end, and the distal base disk 1121 is fixedly connected to or integrally formed with the support 140. For example, the distal ends of the two structural backbone guide tube bundles 113, 113' are distributed at the distal base disk 1121 along the circumference as one bundle or distributed within the circle. It should be appreciated that the distal ends of the two structural backbone guide tube bundles 113, 113' may also be distributed at the distal base disk 1121 along the rectangular periphery as one bundle or distributed within the rectangle. In some embodiments, the proximal base disk 1111, 1111' or the distal base disk 1121 may directly form part of the support 140. In some embodiments, as shown in Fig. 17, at least two drive transmission mechanisms 130, 130' are respectively disposed at the proximal end and the distal end of the support 140, with the output end of each drive transmission mechanism 130, 130' being respectively connected to the corresponding second proximal stop disks 1114, 1114'. The at least two drive transmission mechanisms 130, 130' respectively drive the second proximal stop disks 1114, 1114' of the at least two proximal continua 111, 111' to move so as to drive the first proximal stop disks 1112, 1112' to turn, such that the proximal structural backbones 1116, 1116' of the at least two proximal continua 111, 111' are pushed and pulled so as to achieve the bending of the at least two distal continua 112, 112' in a space in different directions.

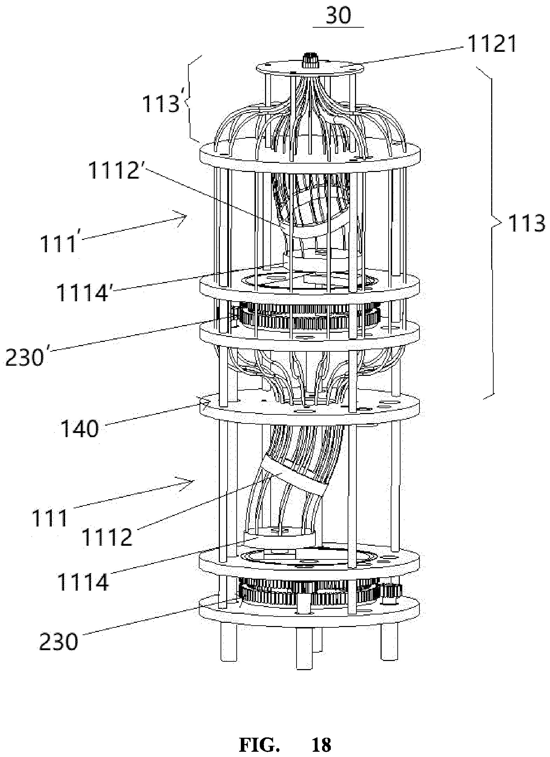

Fig. 18 shows a partial schematic structural diagram of the continuum instrument 30 according to some embodiments of the present disclosure. In some embodiments, as shown in Fig. 18, at least two proximal continua 111, 111' are respectively disposed at the proximal end and the distal end of the support 140, and the distal end of the structural backbone guide tube bundle 113 located at the proximal end passes through the support 140, bypasses the proximal continuum 111', and converges into one bundle at the same distal base disk 1121 together with the distal proximal continuum 111' located at the distal end. In some embodiments, as shown in Fig. 18, the structural backbone guide tube bundle 113 located at the proximal end is distributed in the circumferential direction of the support 140 to form an accommodating space, and the proximal continuum 111' located at the distal end and the structural backbone guide tube bundle 113' are both located in the accommodating space. At least two drive transmission mechanisms 230, 230' are respectively disposed at the proximal end and the distal end of the support 140, with the output end of each of the drive transmission mechanisms 230, 230' being respectively connected to the corresponding second proximal stop disks 1114, 1114'. The at least two drive transmission mechanisms 230, 230' respectively drive the second proximal stop disks 1114, 1114' of at least two proximal continua 111, 111' to move so as to drive the first proximal stop disks 1112, 1112' to turn, which achieves the bending of the at least two distal continua 112, 112' in a space in different directions.

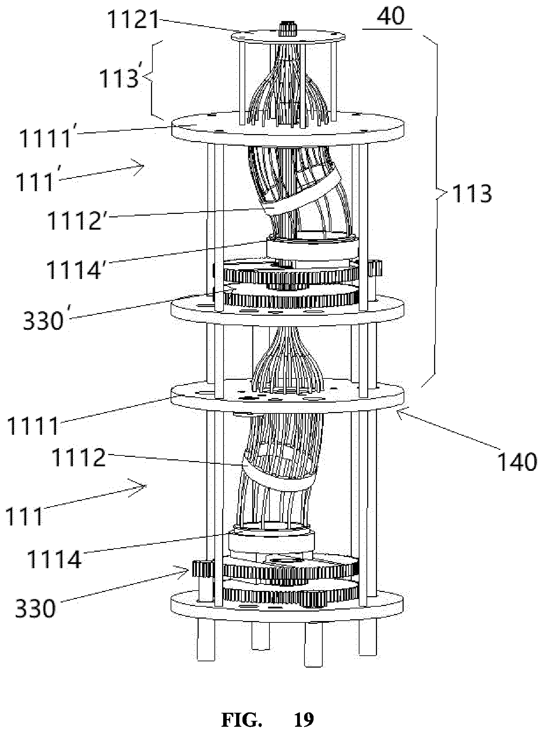

Fig. 19 shows a partial schematic structural diagram of the continuum instrument 40 according to some embodiments of the present disclosure. In some embodiments, as shown in Fig. 19, at least two proximal continua 111, 111' are respectively disposed at the proximal end and the distal end of the support 140, and the proximal ends of the at least two structural backbone guide tube bundles 113, 113' are respectively fixedly connected to the proximal base disks 1111, 1111' of the corresponding proximal continua 111, 111'. The distal end of the structural backbone guide tube bundle 113 located at the proximal end passes through the support 140, and the second proximal stop disk 1114', the first proximal stop disk 1112' and the proximal base disk 1111' of the proximal continuum 111' located at the distal end, and converges into one bundle at the same distal base disk 1121 together with the proximal continuum 111' located at the distal end. At least two drive transmission mechanisms 330, 330' are respectively disposed at the proximal end and the distal end of the support 140, with the output end of each of the drive transmission mechanisms 330, 330' being respectively connected to the corresponding second proximal stop disks 1114, 1114'. The bending of at least two distal continua 112, 112' in different directions in the space may be achieved by means of the at least two drive transmission mechanisms 330, 330'.

In some embodiments, the distal continua 112 in the at least two flexible continuum structures 110 of the continuum instrument 20 (or 30, 40) may have the same or different lengths. It will be appreciated that the distal ends of at least two structural backbone guide tube bundles 113 are converged at the distal base disk 1121. At least two distal continua 112 may be connected in series. For example, the proximal end of the first distal continuum distally extends from the distal base disk 1121 and is fixedly connected to the distal stop disk, the distal base disk of the second distal continuum is connected to or the same as the distal stop disk of the first distal continuum, and the distal end of the second distal continuum may be fixedly connected to the distal stop disk. Thus, the at least two drive transmission mechanisms 130 (or 230, 330) respectively drive the at least two second proximal stop disks 1114 to move to respectively drive the at least two proximal continua 111 to move, so that the distal continua 112 is bent so as to increase the degree of freedom of the distal continua 112 are thus improve the flexibility of the continuum instrument.

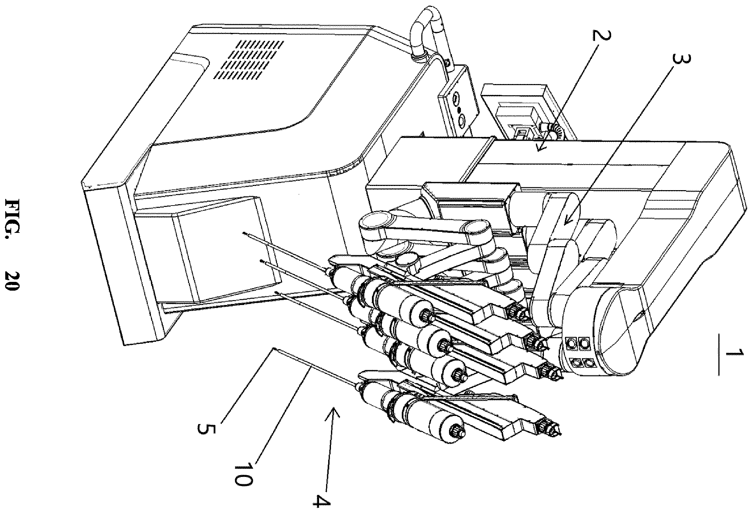

In some embodiments, the present disclosure further provides a surgical robot. The surgical robot includes at least one continuum instrument 10 (or 20, 30, 40) in the embodiments described above. Fig. 20 shows a schematic structural diagram of a surgical robot 1 according to some embodiments of the present disclosure. As shown in Fig. 20, in some embodiments, the surgical robot 1 may further include at least one surgical trolley 2, at least one positioning arm 3, and at least one surgical instrument 4. At least one positioning arm 3 is movably disposed on the at least one surgical trolley 2, and at least one surgical instrument 4 is disposed at the distal end of the at least one positioning arm 3. The surgical instrument 4 includes the continuum instrument 10 (or the continuum instrument 20, 30, or 40) and an end device 5 disposed at the distal end of the continuum instrument 10. It should be appreciated that the end device 5 may include a surgical end effector or an endoscope. The position of the continuum instrument may be adjusted by means of adjusting the positioning arm 3, and the posture of the end device 5 may be adjusted by means of the continuum instrument. The continuum instrument is compact in structure and has high reliability and flexibility, and can improve the safety of the surgical robot.

It should be noted that the foregoing description merely includes exemplary embodiments of the present disclosure and the technical principles applied. It will be appreciated by those skilled in the art that the present disclosure is not limited to the particular embodiments herein, and various obvious changes, readjustments and substitutions can be made by those skilled in the art without departing from the scope of protection of the present disclosure. Therefore, although the present disclosure has been described in detail with reference to the above embodiments, the present disclosure is not limited merely to the above embodiments, and can also include more other equivalent embodiments without departing from the concept of the present disclosure, and the scope of protection of the present disclosure is determined by the scope of the appended claims.

## Claims

1. A continuum instrument, comprising:
at least one proximal continuum comprising a proximal base disk, a first proximal stop disk, a second proximal stop disk, a plurality of proximal structural backbones, and a plurality of proximal drive backbones, proximal ends of the plurality of proximal drive backbones being fixedly connected to the second proximal stop disk, the plurality of proximal drive backbones passing through the first proximal stop disk, and distal ends of the plurality of proximal drive backbones being fixedly connected to the proximal base disk; and
at least one distal continuum comprising a distal stop disk and a plurality of distal structural backbones, the plurality of distal structural backbones being connected to or integrally formed with the plurality of proximal structural backbones, and distal ends of the plurality of distal structural backbones being fixedly connected to the distal stop disk.

2. The continuum instrument according to claim 1, further comprising:
a drive transmission mechanism, an output end of the drive transmission mechanism being connected to the second proximal stop disk and for driving the second proximal stop disk to move, such that the first proximal stop disk is driven to turn by means of the proximal drive backbones, so as to drive the distal continuum to bend by means of the proximal structural backbones and the distal structural backbones.

3. The continuum instrument according to claim 2, wherein the output end of the drive transmission mechanism comprises a sliding pin, the second proximal stop disk comprises a guide hole, and the sliding pin is disposed in the guide hole and is connected to the second proximal stop disk by means of at least one of a cylindrical pair, a moving pair and a rotating pair; or
the output end of the drive transmission mechanism comprises an annular shaft, which is sleeved on the outer periphery of the second proximal stop disk and is connected to the second proximal stop disk by means of at least one of a cylindrical pair, a moving pair and a rotating pair.

4. The continuum instrument according to claim 1, further comprising:
a drive connection part comprising at least one joint, the at least one joint including at least one of a cylindrical pair, a moving pair and a rotating pair, a distal end of the at least one joint being connected to the second proximal stop disk by means of at least one of a cylindrical pair, a moving pair, a rotating pair and fixed connection, and a proximal end of the at least one joint forming an input end.

5. The continuum instrument according to claim 3, wherein
the drive transmission mechanism comprises a planar link mechanism, which comprises a first connecting rod, a second connecting rod, a third connecting rod, a fourth connecting rod, a fifth connecting rod, a first input shaft, and a second input shaft; and
the first connecting rod is fixedly disposed, the first input shaft and the second input shaft are rotatably disposed on the first connecting rod, one end of the second connecting rod is fixedly connected to the first input shaft, the other end of the second connecting rod is hinged to one end of the third connecting rod, one end of the fifth connecting rod is fixedly connected to the second input shaft, the other end of the fifth connecting rod is hinged to one end of the fourth connecting rod, and the other end of the fourth connecting rod is movably connected to the other end of the third connecting rod to form the output end of the drive transmission mechanism.

6. The continuum instrument according to claim 5, wherein the other end of the third connecting rod comprises the annular shaft, and the annular shaft is provided with a groove in a circumferential direction; and
the other end of the fourth connecting rod is disposed in the groove such that the fourth connecting rod and the third connecting rod are rotatably connected to each other.

7. The continuum instrument according to claim 3, wherein the drive transmission mechanism comprises a gear and sliding groove mechanism, which comprises:
a first rotatable member configured to be driven by a first drive member to rotate;
a moving member configured to be driven by the first rotatable member to rotate, a center of rotation of the moving member being offset from a center of rotation of the first rotatable member, the moving member being provided with a first sliding guide portion;
a second rotatable member coaxially disposed with the first rotatable member and configured to be driven by a second drive member to rotate relative to the first rotatable member, the second rotatable member being provided with a second sliding guide portion; and
a sliding assembly slidably connected to the first sliding guide portion and the second sliding guide portion to slide along the first sliding guide portion and the second sliding guide portion, the sliding assembly comprising the output end connected to the second proximal stop disk.

8. The continuum instrument according to claim 7, wherein the moving member comprises a meshing portion for meshing with the first rotatable member; and
an inner peripheral surface of the first rotatable member is provided with inner ring teeth, the meshing portion comprises teeth disposed on an outer peripheral surface of the moving member, and the teeth on the outer peripheral surface of the moving member mesh with the inner ring teeth of the first rotatable member so as to drive the moving member to rotate.

9. The continuum instrument according to claim 8, wherein the gear and sliding groove mechanism further comprises a rotating shaft, which has a proximal end fixedly connected to the moving member and a distal end rotatably connected to the second rotatable member.

10. The continuum instrument according to claim 7, wherein the first sliding guide portion is a first sliding groove, and the second sliding guide portion is a second sliding groove extending in a direction perpendicular to the axis of rotation of the second rotatable member; and
the sliding assembly comprises a sliding pin movably disposed in the first sliding groove and the second sliding groove in a penetrating manner, a distal end of the sliding pin being connected to the second proximal stop disk by means of at least one of a cylindrical pair, a moving pair and a rotating pair.

11. The continuum instrument according to claim 10, wherein the sliding assembly further comprises a sliding block fixedly connected to the sliding pin, the second rotatable member is provided with a sliding rail parallel to the second sliding groove, and the sliding block is slidably disposed on the sliding rail.

12. The continuum instrument according to claim 7, wherein the first sliding guide portion is a first sliding rail, and the second sliding guide portion is a second sliding rail; and
the sliding assembly comprises a first sliding block, a second sliding block and a sliding pin, the first sliding block being slidably disposed on the first sliding rail, the second sliding block being slidably disposed on the second sliding rail, one of the first sliding block and the second sliding block being disposed to be movably connected to the sliding pin, and the other of the first sliding block and the second sliding block being disposed to be fixedly connected to the sliding pin.

13. The continuum instrument according to claim 3, wherein the drive transmission mechanism comprises a gear and rack mechanism, which comprises:
a first rotatable member configured to be driven by a first drive member to rotate;
a second rotatable member coaxially disposed with the first rotatable member and configured to be driven by a second drive member to rotate relative to the first rotatable member, the second rotatable member being provided with a sliding guide portion; and
a moving assembly, at least a portion of the moving assembly being slidably disposed on the sliding guide portion, at least another portion of the moving assembly being disposed to move linearly along with the rotation of the first rotatable member, and a distal end of the moving assembly forming the output end and being connected to the second proximal stop disk.

14. The continuum instrument according to claim 13, wherein the moving assembly comprises a sliding portion and a meshing portion connected to each other, the sliding portion being slidably disposed on the sliding guide portion and then guided by the sliding guide portion to slide linearly relative to the second rotatable member, and the meshing portion being disposed to mesh with the first rotatable member so as to move linearly along with the rotation of the first rotatable member.

15. The continuum instrument according to claim 14, wherein the first rotatable member comprises a meshing gear and a first driven gear coaxially and fixedly connected to each other, the meshing gear being located at a distal side of the first driven gear; and the meshing portion comprises a rack which meshes with the meshing gear.

16. The continuum instrument according to claim 15, wherein the sliding portion comprises a sliding block, the rack is disposed at a proximal end of the sliding block; the sliding guide portion comprises a sliding groove formed in the second rotatable member, the sliding block being slidably disposed in the sliding groove, a proximal side of the sliding block passing through the sliding groove to be fixedly connected to the rack, and the sliding block comprising the output end connected to the second proximal stop disk.

17. The continuum instrument according to claim 1, wherein the distal continuum further comprises a distal base disk, the continuum instrument further comprises a structural backbone guide tube bundle connected between the proximal base disk and the distal base disk, and the proximal structural backbones or the distal structural backbones pass through the proximal base disk and the structural backbone guide tube bundle.

18. The continuum instrument according to claim 17, comprising: at least two proximal continua, at least two distal continua, at least two structural backbone guide tube bundles, and at least two drive transmission mechanisms, wherein the at least two proximal continua are connected in series or in parallel.

19. The continuum instrument according to claim 18, further comprising: a support, wherein
the proximal base disks of the at least two proximal continua are respectively fixedly connected to or integrally formed with the support, and the proximal ends of the at least two structural backbone guide tube bundles are respectively fixedly connected to the proximal base disks of corresponding proximal continua;
the at least two proximal continua comprise a proximal continuum located at a proximal end of the support and a proximal continuum located at a distal end of said support; the structural backbone guide tube bundle corresponding to the proximal continuum located at the proximal end of the support passes through the support and a second proximal stop disk, a first proximal stop disk and a proximal base disk of the proximal continuum located at the distal end of the support and converges into one bundle at the same distal base disk together with the structural backbone guide tube bundle corresponding to the proximal continuum located at a distal end of the support, or the structural backbone guide tube bundle corresponding to the proximal continuum located at the proximal end of the support passes through the support, bypasses the proximal continuum located at the distal end of the support, and converges into one bundle at the same distal base disk together with the structural backbone guide tube bundle corresponding to the proximal continuum located at the distal end of the support;
the distal base disk is fixedly connected to or integrally formed with the support; and
the at least two drive transmission mechanisms are respectively disposed at the proximal end and the distal end of the support, the output end of each of the drive transmission mechanisms is connected to the second proximal stop disk of corresponding proximal continuum to drive the first proximal stop disk of the proximal continuum to turn so as to drive corresponding distal continuum to bend.

20. A surgical robot, comprising at least one surgical trolley, at least one positioning arm, and at least one surgical instrument, wherein
the at least one surgical instrument comprises at least one continuum instrument according to claim 1 and an end device disposed at a distal end of the continuum instrument; and
the at least one positioning arm is movably disposed on the at least one surgical trolley, and the at least one surgical instrument is disposed at a distal end of the at least one positioning arm.
